# EUROPEAN PATENT SPECIFICATION

(11) **EP 4 661 293 B1**
(45) Date of publication and mention of the grant of the patent: **09.12.2026**
(21) Application number: 25211381.6
(22) Date of filing: 28.03.2022
(51) Int. Cl.: A61M 5/315, H03K 3/033, H03K 19/00

(54) **SOUND-DETECTION BASED INJECTION TIME MEASUREMENT SYSTEM FOR DRUG DELIVERY DEVICES**
AUF SCHALLDETEKTION BASIERENDES EINSPRITZZEITMESSSYSTEM FÜR ARZNEIMITTELABGABEVORRICHTUNGEN
SYSTÈME DE MESURE DE TEMPS D'INJECTION BASÉ SUR LA DÉTECTION SONORE POUR DISPOSITIFS D'ADMINISTRATION DE MÉDICAMENT

(30) Priority: 31.03.2021 US 202163168491 P
(43) Date of publication of application: 10.12.2025
(62) Document number(s) of the earlier application(s) in accordance with Art. 76 EPC: 22716745.9 / 4 313 214
(73) Proprietor: AMGEN INC., Thousand Oaks, CA 91320-1799 (US)
(72) Inventor: JIN, Huixing, Thousand Oaks, 91320-1799 (US); JAZAYERI, Julian, Thousand oaks, 91320-1799 (US); VALENZUELA, Sergio Giovanni, Thousand Oaks, 91320-1799 (US); SELVE, Ryan David, Thousand Oaks, 91320-1799 (US); PARAMANANDAM, Sivakumar, Thousand Oaks, 91320-1799 (US)
(74) Representative: J A Kemp LLP

(56) References cited:
- EP-A1- 3 381 493
- US-A1- 2016 166 766

## Description

### CROSS-REFERENCE TO RELATED APPLICATION

Priority is claimed to United States Provisional Patent Application No. 63/168,491, filed March 31, 202.

### FIELD OF DISCLOSURE

The present disclosure generally relates to drug delivery devices, and, more particularly, to sound detection-based approaches for determining injection time durations for drug delivery devices.

### BACKGROUND

Drugs can be administered through the use of drug delivery devices such as autoinjectors or on-body injectors (also referred to as wearable injectors). Autoinjectors and on-body injectors may be used to help automate the injection and delivery or administration process, thereby simplifying the process for certain patient groups or sub-groups for which use of the syringe/vial combination or pre-filled syringe systems would be disadvantageous, whether because of physiological or psychological impediments.

When testing drug delivery autoinjector devices during manufacturing, injection time is often measured as the time required for the autoinjector to deliver the volume of drug contained therein. Patients rely on consistent injection times to prevent premature lifting and/or removal of the autoinjector during injection. For example, if the injection time is longer than an expected time, the patient may think the injection is completed and prematurely remove the autoinjector device prior to complete administration. Injection time may also be an important process control parameter that is used for release testing. During this validation process, it is important that a detection circuit represents the actual variation of the measured autoinjector as opposed to artifacts of the measuring system. In some examples, high speed camera-based imaging systems are used for this purpose. These systems may capture an image of the entire autoinjector during the injection process. At a later time, the images are reviewed by an operator on a frame-by-frame basis to identify and measure the total injection time. However, such systems involve extensive human interpretation and may be susceptible to human error and/or bias relating to when the injection is actually completed.

US 2016/166766 and EP 3 381 493 describe prior art devices for monitoring drug delivery.

As described in more detail below, the present disclosure sets forth systems and methods for sound detection based injection measurement embodying advantageous alternatives to existing systems and methods, and that may address one or more of the challenges or needs mentioned herein, as well as provide other benefits and advantages.

### SUMMARY

In accordance with a first aspect, a real-time drug injection time duration measurement system is provided that includes a sound transducer, a waveform shaping circuit operably coupled with the sound transducer, a pulse clamping circuit operably coupled with the waveform shaping circuit, and a timer operably coupled with the pulse clamping circuit. The sound transducer is adapted to detect a first sound associated with drug delivery initiation and a second sound associated with drug delivery completion and is further adapted to convert the detected first and second sounds into a first electronic waveform and a second electronic waveform, respectively. The waveform shaping circuit is adapted to convert the first and second waveforms into a first pulse and a second pulse, respectively. The pulse clamping circuit is adapted to trigger upon receiving the first pulse and generate a regenerated pulse. The timer is adapted to initiate upon receiving the regenerated pulse and stop upon receiving a subsequent regenerated pulse via the pulse clamping circuit.

In some examples, the waveform shaping circuit may further filter high-pitched signals generated by the first electronic waveform and the second electronic waveform. Further, in some examples, the pulse clamping circuit may generate a pulse output having a designated width corresponding to a specified duration of time to prevent the timer from receiving the subsequent regenerated pulse until the specified duration of time has elapsed. In some examples, the specified duration of time may be approximately 300 milliseconds. In some examples, the pulse clamping circuit includes a mono-stable vibrator that selectively switches between a stable mode and a non-stable mode. In these examples, in the non-stable mode, the pulse clamping circuit is non-responsive to subsequent inputs until completion of the specified duration of time. In these and other examples, the specified duration of time may have a longer duration than a duration of the first detected sound.

In accordance with a second aspect, an approach measuring in real-time an injection time duration measurement for a drug delivery device is provided that includes detecting, via a sound transducer, a first sound associated with initiation of the drug delivery device. The first detected sound is converted into a first electronic waveform. A waveform shaping circuit operably coupled with the sound transducer converts the first waveform into a first pulse. A pulse clamping circuit operably coupled with the waveform shaping circuit is triggered upon receiving the first pulse. The pulse clamping circuit then generates a regenerated pulse and a timer operably coupled with the pulse clamping circuit is initiated upon receiving the regenerated pulse. The timer is terminated upon receiving a subsequent regenerated pulse.

### BRIEF DESCRIPTION OF THE DRAWINGS

The above needs are at least partially met through provision of the systems and approaches for sound detection based injection duration measurement described in the following detailed description, particularly when studied in conjunction with the drawings, wherein:
Fig. 1 illustrates a block diagram of an example real-time sound detection based injection duration measurement system in accordance with various embodiments;
Fig. 2 illustrates an example display depicting an example waveform capture of the example real-time sound detection based injection duration measurement system of Fig. 1 in accordance with various embodiments;
Fig. 3 illustrates the example display of Fig. 2 depicting an example subsequent waveform capture of the example real-time sound detection based injection duration measurement system in accordance with various embodiments;
Fig. 4 illustrates a circuit diagram of the example real-time sound detection based injection duration measurement system of Figs. 1-3 in accordance with various embodiments.

Skilled artisans will appreciate that elements in the figures are illustrated for simplicity and clarity and have not necessarily been drawn to scale. For example, the dimensions and/or relative positioning of some of the elements in the figures may be exaggerated relative to other elements to help to improve understanding of various embodiments of the present invention. Also, common but well-understood elements that are useful or necessary in a commercially feasible embodiment are often not depicted in order to facilitate a less obstructed view of these various embodiments. It will further be appreciated that certain actions and/or steps may be described or depicted in a particular order of occurrence while those skilled in the art will understand that such specificity with respect to sequence is not actually required. It will also be understood that the terms and expressions used herein have the ordinary technical meaning as is accorded to such terms and expressions by persons skilled in the technical field as set forth above except where different specific meanings have otherwise been set forth herein.

### DETAILED DESCRIPTION

Generally speaking, pursuant to these various embodiments, a real-time approach for sound detection based injection duration measurement is provided. In some examples, the system may use a transducer and a number of circuits to accurately and reliably measure injection times attuned to a user. Further, the system measures injection times of the sample drug delivery device in real-time to allow for automation into a testing workflow used to inspect and ensure the device is ready for distribution to patients and/or healthcare facilities responsible for drug administration.

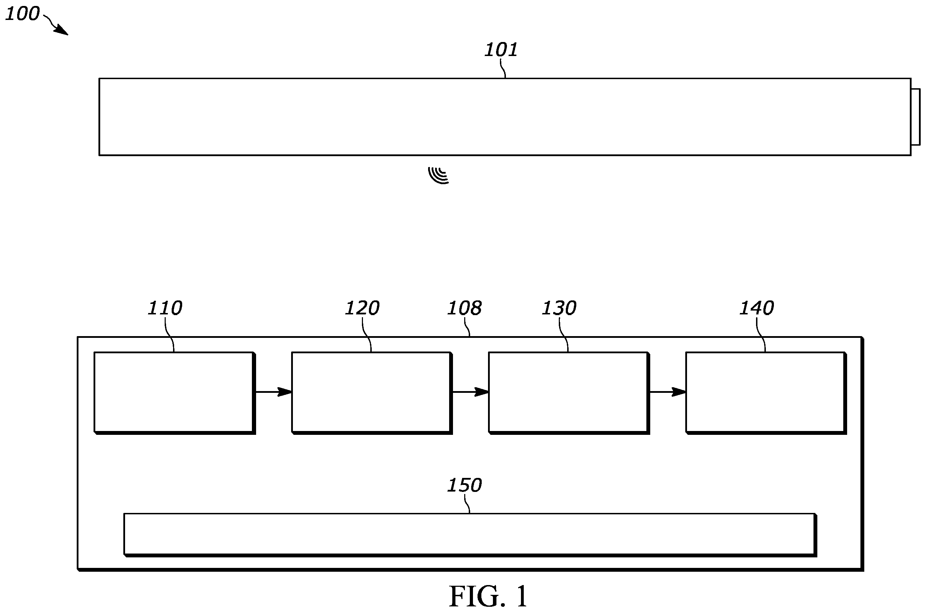

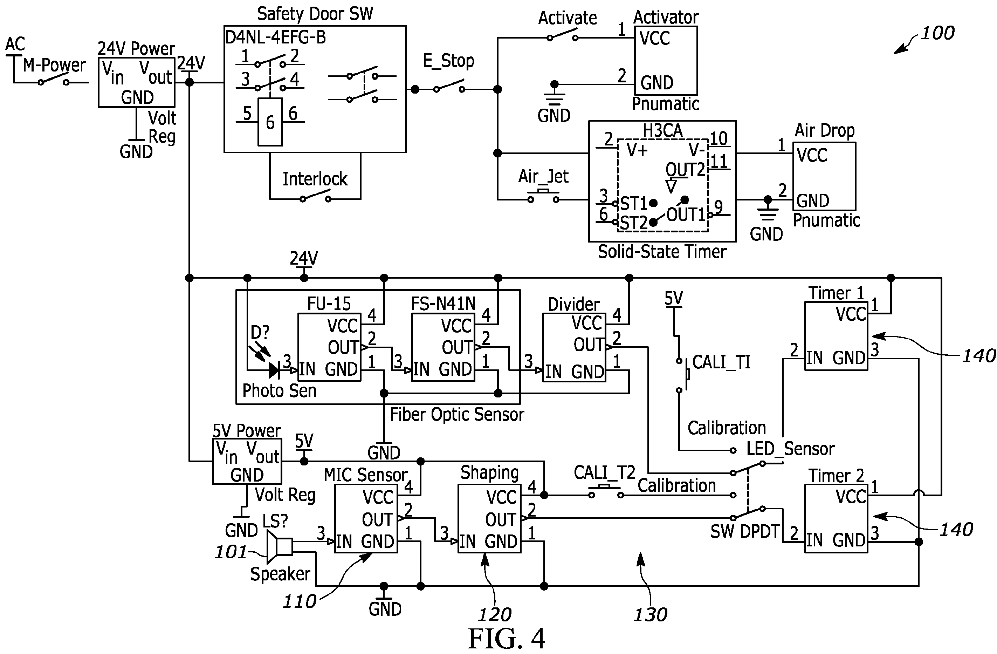

As illustrated in Figs. 1 and 4, a real-time drug injection time duration measurement system 100 is provided for use with a drug delivery device 101 (e.g., an autoinjector). The system 100 includes a computing system 108 having a sound transducer 110, a waveform shaping circuit 120 operably coupled with the sound transducer 110, a pulse clamping circuit 130 operably coupled with the waveform shaping circuit 120, and a timer/counter 140 operably coupled with the pulse clamping circuit 130. The computing system 108 may further include an interface 150 in the form of a display to provide a visual representation of the received signal and/or processing being applied thereto. It is appreciated that the system 100 may include any number of additional components such as processors, memory modules, programs, executable instructions, and the like to assist in operation. Further, the computing system 108 may include any number of mechanical and electromechanical components, subcomponents, systems, power sources, measuring devices, processors, controllers, and the like to operate in an autonomous or semi-autonomous manner.

It is appreciated that the autoinjector 101 is just one example of a suitable drug delivery device that may be measured in real-time. The autoinjector 101 may include an actuation mechanism 102 (e.g., a button) to cause the autoinjector 101 to commence injection and drug delivery. Notably, the autoinjector 101 is equipped with an auditory notification function that notifies the user about important operational condition changes. For example, the autoinjector 101 may generate an audio signal in the form of a short clicking sound that lasts a short duration (e.g., between approximately 5 milliseconds and approximately 50 milliseconds) and is issued just prior to the start and end of injection. These audio signals may be generated via any number of mechanical and/or electromechanical components and are captured by the computing system 108 and used as triggers to control the counter 140 to allow a time measurement system to measure the injection time.

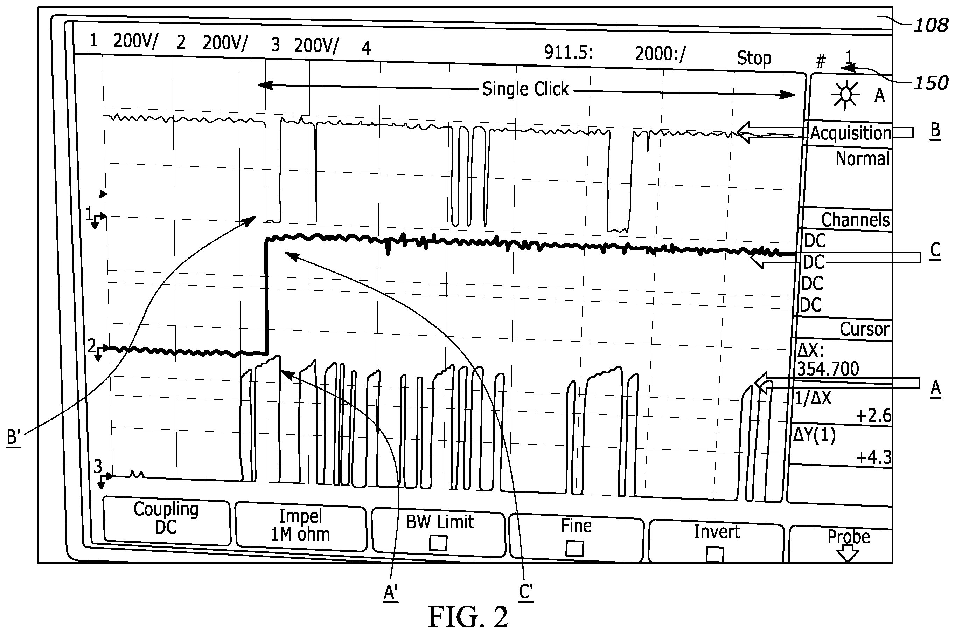

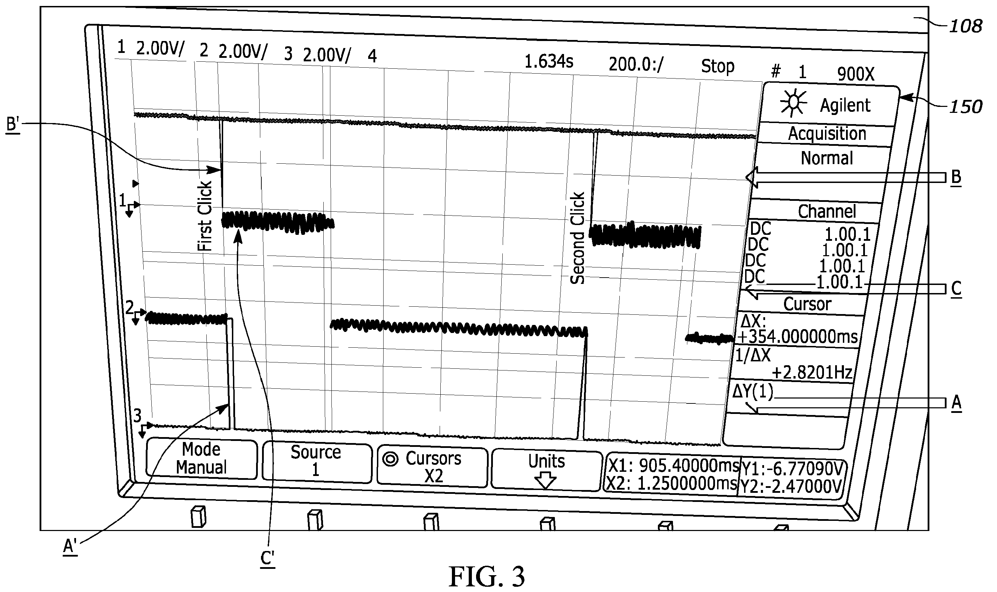

In some examples, the sound transducer 110 is in the form of a sensor capable of detecting the audio signal generated by the autoinjector 101 and converting it to an electronic waveform depicted by line "A" in Figs. 2 and 3. More specifically, in Fig. 2, a single detected audio signal corresponding to initiation of injection is illustrated on the display 150, and in Fig. 3, first and second audio signals corresponding to initiation and completion of injection, respectively are illustrated on the display 150. The waveform shaping circuit 120, depicted by line "B" in Figs. 2 and 3, filters any high-pitched glitches or undesired frequencies received by the sound transducer 110 and converts the waveform (which is continuously varying) into a string of closely-following high or low valued pulses (labeled B').

In some examples, the pulse clamping circuit 130 may be in the form of a mono-stable vibrator that is triggered to generate a regenerated pulse output having a width that is determined by specified design parameters. Notably, a mono-stable vibrator may change between stable and non-stable modes in response to a specific triggering pulse. Upon entering the non-stable mode, the pulse clamping circuit 130 enters into a "recovering" phase whereby it is non-responsive (i.e., self-locked) to any subsequent inputs until the end of a specified duration or time constant. Upon completion of the time constant, the pulse clamping circuit 130 may resume detection capability.

In some approaches, the time constant used by the pulse clamping circuit 130 is significantly longer than the received input pulse string generated upon the sound transducer 110 sensing the first audio signal corresponding to initiation of the actuator 101. For example, while the audio signal generated by the actuator 101 may have a duration between approximately 5 milliseconds and approximately 50 milliseconds, the time constant used by the pulse clamping circuit 130 may have a duration of approximately 300ms to ensure the pulse clamping circuit 130 only reacts to the first incoming pulse and clamps (or blocks) subsequent incoming pulses. This operation assures a single pulse is generated from the pulse string of each audio signal. As illustrated in Figs. 2 and 3, the pulse clamping circuit 130 generates a stable signal depicted by line "C" having pulses depicted by " C' " that correspond to its activation.

The timer 140 receives and uses the regenerated pulse that is generated by the pulse clamping circuit 130 as a triggering signal to selectively initiate or stop the time duration measurement. Accordingly, the timer 140 generates a reliable and accurate measurement of the injection time that is automated and not subject to artifacts generated by the audio waveform signal generated by the sound transducer 110.

In some examples, an approach for conducting the injection time duration measurement for the autoinjector 101 in real time includes first detecting, using the sound transducer 110, a first sound associated with initiation of the autoinjector. The first detected sound is converted into a first electronic waveform. The waveform shaping circuit 120 converts the first waveform into a first pulse. The pulse clamping circuit 130 is triggered upon receiving the first pulse. The pulse clamping circuit 130 then generates a regenerated pulse and the timer 140 is initiated upon receiving the regenerated pulse. The timer 140 is terminated upon receiving a subsequent regenerated pulse. It is appreciated that the subsequent regenerated pulse may also be generated and converted into a waveform by the sound transducer 110 and subsequently converted into a second pulse by the waveform shaping circuit 120.

With reference to Fig. 2, example waveform capture operation of the computing system 108 is illustrated on the display 150. As depicted by line A, the sound transducer receives a string of pulses which correspond to a single click of the audio signal generated by the autoinjector 101. In this example, a time base of 2 milliseconds per division is provided, and as such, a single click generated by the audio signal is approximately 8 divisions or 16 milliseconds long. As previously noted, the pulse clamping circuit 130 is only responsive to the first input pulse and does not react to subsequent pulses generated by the sound transducer 110 until completion of its recovery phase.

With reference to Fig. 3, example waveform capture operation of the computing system 108 is illustrated on the display 150 with a time base of 200 milliseconds per division to allow for visualization of successive clicks and corresponding audio signals generated by the autoinjector 101. In this example, the injection time from the first audio signal to the second audio signal is six divisions, which corresponds to approximately 1.2 seconds. Further, in this example, the time constant of the pulse clamping circuit 130 is tuned to be approximately 1.5 divisions or approximately 300 milliseconds, and as such, the pulse clamping circuit 130 will be fully recovered and ready to sense subsequent audio signals before receiving the second audio signal corresponding to completion of injection.

The timer 140 measures the elapsed time between the regenerated pulses generated by the pulse clamping circuit 130, which corresponds to initiation and completion of drug delivery. By measuring the time between the audible signals, the measurement is attuned with the same cues that the user receives for the beginning and the end of injection. As previously noted, this measurement can be made in real-time and is not subject to retrospective human interpretation of captured footage. The approaches described herein are cost effective and less prone to operator error as compared with conventional systems that require an operator to make a judgment call as to when initiation and/or completion of drug delivery has occurred. Further, by measuring these signals in real-time, production efficiencies may be captured, thereby allowing automation and integration with a series of tests.

In the event the timing data is different than an accepted value or falls outside an accepted range of values, additional inspection may be needed. More specifically, a differing time may be indicative that the autoinjector 101 (and/or those autoinjectors manufactured in the same group or lot) is out of spec with the expected parameters. As such, device and component inspection may be needed to ensure all components of the autoinjector 101 (and/or other autoinjectors assembled in the same group or lot) were manufactured and assembled within tolerances. It is appreciated that in some examples, the system 100 may perform a "destructive" test, meaning the actual autoinjector 101 being tested is not shipped to consumers for use. In such examples, the results of the test may be extrapolated to other devices in the same group or lot.

So configured, the system described herein ensures the autoinjector is delivering the drug within an acceptable time frame. The autoinjector may include instructions for use that indicate the patient should hear beginning and ending notification clicking sounds, and as such, the system may provide a confirmation that the measured injection time matches the time set forth in the use instructions.

The above description describes various devices, assemblies, components, subsystems and methods for use related to a drug delivery device. The devices, assemblies, components, subsystems, methods or drug delivery devices can further comprise or be used with a drug including but not limited to those drugs identified below as well as their generic and biosimilar counterparts. The term drug, as used herein, can be used interchangeably with other similar terms and can be used to refer to any type of medicament or therapeutic material including traditional and non-traditional pharmaceuticals, nutraceuticals, supplements, biologics, biologically active agents and compositions, large molecules, biosimilars, bioequivalents, therapeutic antibodies, polypeptides, proteins, small molecules and generics. Non-therapeutic injectable materials are also encompassed. The drug may be in liquid form, a lyophilized form, or in a reconstituted from lyophilized form. The following example list of drugs should not be considered as all-inclusive or limiting.

The drug will be contained in a reservoir. In some instances, the reservoir is a primary container that is either filled or pre-filled for treatment with the drug. The primary container can be a vial, a cartridge, a carboy, a bag, a pre-filled syringe, or any suitable drug container, drug product container, drug substance container, or other drug-related container.

In some embodiments, the reservoir of the drug delivery device may be filled with or the device can be used with colony stimulating factors, such as granulocyte colony-stimulating factor (G-CSF). Such G-CSF agents include but are not limited to Neulasta^{®} (pegfilgrastim, pegylated filgastrim, pegylated G-CSF, pegylated hu-Met-G-CSF) and Neupogen^{®} (filgrastim, G-CSF, hu-MetG-CSF).

In other embodiments, the drug delivery device may contain or be used with an erythropoiesis stimulating agent (ESA), which may be in liquid or lyophilized form. An ESA is any molecule that stimulates erythropoiesis. In some embodiments, an ESA is an erythropoiesis stimulating protein. As used herein, "erythropoiesis stimulating protein" means any protein that directly or indirectly causes activation of the erythropoietin receptor, for example, by binding to and causing dimerization of the receptor. Erythropoiesis stimulating proteins include erythropoietin and variants, analogs, or derivatives thereof that bind to and activate erythropoietin receptor; antibodies that bind to erythropoietin receptor and activate the receptor; or peptides that bind to and activate erythropoietin receptor. Erythropoiesis stimulating proteins include, but are not limited to, Epogen^{®} (epoetin alfa), Aranesp^{®} (darbepoetin alfa), Dynepo^{®} (epoetin delta), Mircera^{®} (methyoxy polyethylene glycol-epoetin beta), Hematide^{®}, MRK-2578, INS-22, Retacrit^{®} (epoetin zeta), Neorecormon^{®} (epoetin beta), Silapo^{®} (epoetin zeta), Binocrit^{®} (epoetin alfa), epoetin alfa Hexal, Abseamed^{®} (epoetin alfa), Ratioepo^{®} (epoetin theta), Eporatio^{®} (epoetin theta), Biopoin^{®} (epoetin theta), epoetin alfa, epoetin beta, epoetin iota, epoetin omega, epoetin delta, epoetin zeta, epoetin theta, and epoetin delta, pegylated erythropoietin, carbamylated erythropoietin, as well as the molecules or variants or analogs thereof.

Among particular illustrative proteins are the specific proteins set forth below, including fusions, fragments, analogs, variants or derivatives thereof: OPGL specific antibodies, peptibodies, related proteins, and the like (also referred to as RANKL specific antibodies, peptibodies and the like), including fully humanized and human OPGL specific antibodies, particularly fully humanized monoclonal antibodies; Myostatin binding proteins, peptibodies, related proteins, and the like, including myostatin specific peptibodies; IL-4 receptor specific antibodies, peptibodies, related proteins, and the like, particularly those that inhibit activities mediated by binding of IL-4 and/or IL-13 to the receptor; Interleukin 1-receptor 1 ("IL1-R1") specific antibodies, peptibodies, related proteins, and the like; Ang2 specific antibodies, peptibodies, related proteins, and the like; NGF specific antibodies, peptibodies, related proteins, and the like; CD22 specific antibodies, peptibodies, related proteins, and the like, particularly human CD22 specific antibodies, such as but not limited to humanized and fully human antibodies, including but not limited to humanized and fully human monoclonal antibodies, particularly including but not limited to human CD22 specific IgG antibodies, such as, a dimer of a human-mouse monoclonal hLL2 gamma-chain disulfide linked to a human-mouse monoclonal hLL2 kappa-chain, for example, the human CD22 specific fully humanized antibody in Epratuzumab, CAS registry number 501423-23-0; IGF-1 receptor specific antibodies, peptibodies, and related proteins, and the like including but not limited to anti-IGF-1R antibodies; B-7 related protein 1 specific antibodies, peptibodies, related proteins and the like ("B7RP-1" and also referring to B7H2, ICOSL, B7h, and CD275), including but not limited to B7RP-specific fully human monoclonal IgG2 antibodies, including but not limited to fully human IgG2 monoclonal antibody that binds an epitope in the first immunoglobulin-like domain of B7RP-1, including but not limited to those that inhibit the interaction of B7RP-1 with its natural receptor, ICOS, on activated T cells; IL-15 specific antibodies, peptibodies, related proteins, and the like, such as, in particular, humanized monoclonal antibodies, including but not limited to HuMax IL-15 antibodies and related proteins, such as, for instance, 146B7; IFN gamma specific antibodies, peptibodies, related proteins and the like, including but not limited to human IFN gamma specific antibodies, and including but not limited to fully human anti-IFN gamma antibodies; TALL-1 specific antibodies, peptibodies, related proteins, and the like, and other TALL specific binding proteins; Parathyroid hormone ("PTH") specific antibodies, peptibodies, related proteins, and the like; Thrombopoietin receptor ("TPO-R") specific antibodies, peptibodies, related proteins, and the like;Hepatocyte growth factor ("HGF") specific antibodies, peptibodies, related proteins, and the like, including those that target the HGF/SF:cMet axis (HGF/SF:c-Met), such as fully human monoclonal antibodies that neutralize hepatocyte growth factor/scatter (HGF/SF); TRAIL-R2 specific antibodies, peptibodies, related proteins and the like; Activin A specific antibodies, peptibodies, proteins, and the like; TGF-beta specific antibodies, peptibodies, related proteins, and the like; Amyloid-beta protein specific antibodies, peptibodies, related proteins, and the like; c-Kit specific antibodies, peptibodies, related proteins, and the like, including but not limited to proteins that bind c-Kit and/or other stem cell factor receptors; OX40L specific antibodies, peptibodies, related proteins, and the like, including but not limited to proteins that bind OX40L and/or other ligands of the OX40 receptor; Activase^{®} (alteplase, tPA); Aranesp^{®} (darbepoetin alfa); Epogen^{®} (epoetin alfa, or erythropoietin); GLP-1, Avonex^{®} (interferon beta-1a); Bexxar^{®} (tositumomab, anti-CD22 monoclonal antibody); Betaseron^{®} (interferon-beta); Campath^{®} (alemtuzumab, anti-CD52 monoclonal antibody); Dynepo^{®} (epoetin delta); Velcade^{®} (bortezomib); MLN0002 (anti- a4ß7 mAb); MLN1202 (anti-CCR2 chemokine receptor mAb); Enbrel^{®} (etanercept, TNF-receptor /Fc fusion protein, TNF blocker); Eprex^{®} (epoetin alfa); Erbitux^{®} (cetuximab, anti-EGFR / HER1 / c-ErbB-1); Genotropin^{®} (somatropin, Human Growth Hormone); Herceptin^{®} (trastuzumab, anti-HER2/neu (erbB2) receptor mAb); Humatrope^{®} (somatropin, Human Growth Hormone); Humira^{®} (adalimumab); Vectibix^{®} (panitumumab), Xgeva^{®} (denosumab), Prolia^{®} (denosumab), Enbrel^{®} (etanercept, TNF-receptor /Fc fusion protein, TNF blocker), Nplate^{®} (romiplostim), rilotumumab, ganitumab, conatumumab, brodalumab, insulin in solution; Infergen^{®} (interferon alfacon-1); Natrecor^{®} (nesiritide; recombinant human B-type natriuretic peptide (hBNP); Kineret^{®} (anakinra); Leukine^{®} (sargamostim, rhuGM-CSF); LymphoCide^{®} (epratuzumab, anti-CD22 mAb); Benlysta^{™} (lymphostat B, belimumab, anti-BlyS mAb); Metalyse^{®} (tenecteplase, t-PA analog); Mircera^{®} (methoxy polyethylene glycol-epoetin beta); Mylotarg^{®} (gemtuzumab ozogamicin); Raptiva^{®} (efalizumab); Cimzia^{®} (certolizumab pegol, CDP 870); Soliris^{™} (eculizumab); pexelizumab (anti-C5 complement); Numax^{®} (MEDI-524); Lucentis^{®} (ranibizumab); Panorex^{®} (17-1A, edrecolomab); Trabio^{®} (lerdelimumab); TheraCim hR3 (nimotuzumab); Omnitarg (pertuzumab, 2C4); Osidem^{®} (IDM-1); OvaRex^{®} (B43.13); Nuvion^{®} (visilizumab); cantuzumab mertansine (huC242-DM1); NeoRecormon^{®} (epoetin beta); Neumega^{®} (oprelvekin, human interleukin-11); Orthoclone OKT3^{®} (muromonab-CD3, anti-CD3 monoclonal antibody); Procrit^{®} (epoetin alfa); Remicade^{®} (infliximab, anti-TNFα monoclonal antibody); Reopro^{®} (abciximab, anti-GP IIb/IIia receptor monoclonal antibody); Actemra^{®} (anti-IL6 Receptor mAb); Avastin^{®} (bevacizumab), HuMax-CD4 (zanolimumab); Rituxan^{®} (rituximab, anti-CD20 mAb); Tarceva^{®} (erlotinib); Roferon-A^{®}-(interferon alfa-2a); Simulect^{®} (basiliximab); Prexige^{®} (lumiracoxib); Synagis^{®} (palivizumab); 146B7-CHO (anti-IL15 antibody, see U.S. Patent No. 7,153,507); Tysabri^{®} (natalizumab, anti-α4integrin mAb); Valortim^{®} (MDX-1303, anti-B. anthracis protective antigen mAb); ABthrax^{™}; Xolair^{®} (omalizumab); ETI211 (anti-MRSA mAb); IL-1 trap (the Fc portion of human IgG1 and the extracellular domains of both IL-1 receptor components (the Type I receptor and receptor accessory protein)); VEGF trap (Ig domains of VEGFR1 fused to IgG1 Fc); Zenapax^{®} (daclizumab); Zenapax^{®} (daclizumab, anti-IL-2Rα mAb); Zevalin^{®} (ibritumomab tiuxetan); Zetia^{®} (ezetimibe); Orencia^{®} (atacicept, TACI-Ig); anti-CD80 monoclonal antibody (galiximab); anti-CD23 mAb (lumiliximab); BR2-Fc (huBR3 / huFc fusion protein, soluble BAFF antagonist); CNTO 148 (golimumab, anti-TNFα mAb); HGS-ETR1 (mapatumumab; human anti-TRAIL Receptor-1 mAb); HuMax-CD20 (ocrelizumab, anti-CD20 human mAb); HuMax-EGFR (zalutumumab); M200 (volociximab, anti-α5β1 integrin mAb); MDX-010 (ipilimumab, anti-CTLA-4 mAb and VEGFR-1 (IMC-18F1); anti-BR3 mAb; anti-C. difficile Toxin A and Toxin B C mAbs MDX-066 (CDA-1) and MDX-1388); anti-CD22 dsFv-PE38 conjugates (CAT-3888 and CAT-8015); anti-CD25 mAb (HuMax-TAC); anti-CD3 mAb (NI-0401); adecatumumab; anti-CD30 mAb (MDX-060); MDX-1333 (anti-IFNAR); anti-CD38 mAb (HuMax CD38); anti-CD40L mAb; anti-Cripto mAb; anti-CTGF Idiopathic Pulmonary Fibrosis Phase I Fibrogen (FG-3019); anti-CTLA4 mAb; anti-eotaxin1 mAb (CAT-213); anti-FGF8 mAb; anti-ganglioside GD2 mAb; anti-ganglioside GM2 mAb; anti-GDF-8 human mAb (MYO-029); anti-GM-CSF Receptor mAb (CAM-3001); anti-HepC mAb (HuMax HepC); anti-IFNα mAb (MEDI-545, MDX-1103); anti-IGF1R mAb; anti-IGF-1R mAb (HuMax-Inflam); anti-IL12 mAb (ABT-874); anti-IL12/IL23 mAb (CNTO 1275); anti-IL13 mAb (CAT-354); anti-IL2Ra mAb (HuMax-TAC); anti-IL5 Receptor mAb; anti-integrin receptors mAb (MDX-018, CNTO 95); anti-IP10 Ulcerative Colitis mAb (MDX-1100); BMS-66513; anti-Mannose Receptor/hCGβ mAb (MDX-1307); anti-mesothelin dsFv-PE38 conjugate (CAT-5001); anti-PD1mAb (MDX-1106 (ONO-4538)); anti-PDGFRα antibody (IMC-3G3); anti-TGFß mAb (GC-1008); anti-TRAIL Receptor-2 human mAb (HGS-ETR2); anti-TWEAK mAb; anti-VEGFR/Flt-1 mAb; and anti-ZP3 mAb (HuMax-ZP3).

In some embodiments, the drug delivery device may contain or be used with a sclerostin antibody, such as but not limited to romosozumab, blosozumab, or BPS 804 (Novartis) and in other embodiments, a monoclonal antibody (IgG) that binds human Proprotein Convertase Subtilisin/Kexin Type 9 (PCSK9). Such PCSK9 specific antibodies include, but are not limited to, Repatha^{®} (evolocumab) and Praluent^{®} (alirocumab). In other embodiments, the drug delivery device may contain or be used with rilotumumab, bixalomer, trebananib, ganitumab, conatumumab, motesanib diphosphate, brodalumab, vidupiprant or panitumumab. In some embodiments, the reservoir of the drug delivery device may be filled with or the device can be used with IMLYGIC^{®} (talimogene laherparepvec) or another oncolytic HSV for the treatment of melanoma or other cancers including but are not limited to OncoVEXGALV/CD; OrienX010; G207, 1716; NV1020; NV12023; NV1034; and NV1042. In some embodiments, the drug delivery device may contain or be used with endogenous tissue inhibitors of metalloproteinases (TIMPs) such as but not limited to TIMP-3. Antagonistic antibodies for human calcitonin gene-related peptide (CGRP) receptor such as but not limited to erenumab and bispecific antibody molecules that target the CGRP receptor and other headache targets may also be delivered with a drug delivery device of the present disclosure. Additionally, bispecific T cell engager (BiTE^{®}) antibodies such as but not limited to half-life extended BiTEs that include an antibody Fc region, BLINCYTO^{®} (blinatumomab) can be used in or with the drug delivery device of the present disclosure. In some embodiments, the drug delivery device may contain or be used with an APJ large molecule agonist such as but not limited to apelin or analogues thereof. In some embodiments, a therapeutically effective amount of an anti-thymic stromal lymphopoietin (TSLP) or TSLP receptor antibody is used in or with the drug delivery device of the present disclosure.

Although the drug delivery devices, assemblies, components, subsystems and methods have been described in terms of exemplary embodiments, they are not limited thereto. The detailed description is to be construed as exemplary only and does not describe every possible embodiment of the present disclosure. Numerous alternative embodiments could be implemented, using either current technology or technology developed after the filing date of this patent that would still fall within the scope of the claims defining the invention(s) disclosed herein.

Those skilled in the art will recognize that a wide variety of modifications, alterations, and combinations can be made with respect to the above described embodiments without departing from the scope of the invention(s) disclosed herein and that such modifications, alterations, and combinations are to be viewed as being within the ambit of the inventive concept(s).

## Claims

1. A real-time drug injection time duration measurement system (100) comprising:
a sound transducer (110) adapted to detect a first sound associated with drug delivery initiation of a drug delivery device and a second sound associated with drug delivery completion of the drug delivery device, the sound transducer further adapted to convert the detected first and second sounds into a first electronic waveform and a second electronic waveform, respectively;
a waveform shaping circuit (120) operably coupled with the sound transducer, the waveform shaping circuit adapted to convert the first and second waveforms into a first pulse and a second pulse, respectively;
a pulse clamping circuit (130) operably coupled with the waveform shaping circuit, the pulse clamping circuit adapted to trigger upon receiving the first pulse and generate a regenerated pulse; and
a timer (140) operably coupled with the pulse clamping circuit, the timer adapted to initiate upon receiving the regenerated pulse and stop upon receiving a subsequent regenerated pulse via the pulse clamping circuit, wherein an injection time of the drug delivery device is measured based on an elapsed time between the regenerated pulse received by the timer and the subsequent regenerated pulse.

2. The real-time drug injection time duration measurement system of claim 1, wherein the waveform shaping circuit is further adapted to filter high-pitched signals generated by the first electronic waveform and the second electronic waveform.

3. The real-time drug injection time duration measurement system of claim 1 or 2, wherein the pulse clamping circuit is adapted to generate a pulse output having a designated width corresponding to a specified duration of time to prevent the timer from receiving the subsequent regenerated pulse until the specified duration of time has elapsed.

4. The real-time drug injection time duration measurement system of claim 3, wherein the specified duration of time is approximately 300 milliseconds.

5. The real-time drug injection time duration measurement system of claim 3 or 4, wherein the pulse clamping circuit comprises a mono-stable vibrator adapted to selectively switch between a stable mode and a non-stable mode, wherein in the non-stable mode, the pulse clamping circuit is non-responsive to subsequent inputs until completion of the specified duration of time.

6. The real-time drug injection time duration measurement system of any one of claims 3-5, wherein the specified duration of time has a longer duration than a duration of the first detected sound.

7. The real-time drug injection time duration measurement system of any one of claims 1-6, wherein the sound transducer comprises a sensor.

8. A method of measuring in real-time an injection time duration measurement for a drug delivery device (101), the method comprising:
detecting, via a sound transducer (110), a first sound associated with drug delivery initiation of the drug delivery device;
converting the first detected sound into a first electronic waveform;
converting, via a waveform shaping circuit (120) operably coupled with the sound transducer, the first waveform into a first pulse;
triggering a pulse clamping circuit (130) operably coupled with the waveform shaping circuit upon receiving the first pulse;
generating, via the pulse clamping circuit, a regenerated pulse;
initiating a timer (140) operably coupled with the pulse clamping circuit upon receiving the regenerated pulse; and
terminating the timer upon receiving a subsequent regenerated pulse,
wherein an injection time of the drug delivery device is measured based on an elapsed time between the regenerated pulse received by the timer and the subsequent regenerated pulse.

9. The method of claim 8, further comprising the steps of:
detecting, via the sound transducer, a second sound associated with drug delivery completion of the drug delivery device;
converting the second detected sound into a second electronic waveform;
converting the second waveform into a second pulse via the waveform shaping circuit; and
generating the subsequent regenerated pulse via the pulse clamping circuit.

10. The method of claim 9, wherein the regenerated pulse generated by the pulse clamping circuit has a designated width corresponding to a specified duration of time to prevent the timer from receiving the subsequent regenerated pulse until the specified duration of time has elapsed.

11. The method of claim 10, wherein the specified duration of time of approximately 300 milliseconds.

12. The method of claim 10 or 11, wherein the step of triggering the pulse clamping circuit further comprises selectively switching, via a mono-stable vibrator, between a stable mode and a non-stable mode, wherein in the non-stable mode, the pulse clamping circuit is non-responsive to subsequent inputs until completion of the specified duration of time.

13. The method of any one of claims 10-12, wherein the specified duration of time has a longer duration than a duration of the first detected sound.

14. The method of any one of claims 8-13, further comprising the step of filtering, via the waveform shaping circuit, at least one high-pitched signal generated by the first electronic waveform and/or the second electronic waveform.

15. The method of any one of claims 8-14, wherein the sound transducer comprises a sensor.

## Patentansprüche

1. System (100) für die Messung der Zeitdauer der Arzneimittelinjektion in Echtzeit umfassend:
einen Schallwandler (110), der eingerichtet ist, um einen ersten Schall, der der Einleitung der Arzneimittelabgabe einer Arzneimittelabgabevorrichtung zugeordnet ist, und einen zweiten Schall, der dem Abschluss der Arzneimittelabgabe der Arzneimittelabgabevorrichtung zugeordnet ist, zu erfassen, wobei der Schallwandler ferner eingerichtet ist, um den erfassten ersten und den erfassten zweiten Schall jeweils in eine erste elektronische Wellenform und eine zweite elektronische Wellenform umzuwandeln;
eine Wellenformkorrekturschaltung (120), die betriebsfähig mit dem Schallwandler gekoppelt ist, wobei die Wellenformkorrekturschaltung eingerichtet ist, um die erste und die zweite Wellenform jeweils in einen ersten Impuls und einen zweiten Impuls umzuwandeln;
eine Impulsklemmschaltung (130), die betriebsfähig mit der Wellenformkorrekturschaltung gekoppelt ist, wobei die Impulsklemmschaltung eingerichtet ist, um nach dem Empfangen des ersten Impulses auszulösen und einen regenerierten Impuls zu erzeugen; und
einen Zeitgeber (140), der betriebsfähig mit der Impulsklemmschaltung gekoppelt ist, wobei der Zeitgeber eingerichtet ist, um bei dem Empfang des regenerierten Impulses zu starten und bei dem Empfang eines nachfolgenden regenerierten Impulses über die Impulsklemmschaltung zu stoppen, wobei eine Injektionszeit der Arzneimittelabgabevorrichtung basierend auf einer verstrichenen Zeit zwischen dem regenerierten Impuls, der von dem Zeitgeber empfangen wird, und dem nachfolgenden regenerierten Impuls gemessen wird.

2. System für die Messung der Zeitdauer der Arzneimittelinjektion in Echtzeit nach Anspruch 1, wobei die Wellenformkorrekturschaltung ferner eingerichtet ist, um hochfrequente Signale zu filtern, die durch die erste elektronische Wellenform und die zweite elektronische Wellenform erzeugt werden.

3. System für die Messung der Zeitdauer der Arzneimittelinjektion in Echtzeit nach Anspruch 1 oder 2, wobei die Impulsklemmschaltung eingerichtet ist, um eine Impulsausgabe zu erzeugen, die eine bestimmte Breite aufweist, die einer spezifizierten Zeitdauer entspricht, um zu verhindern, dass der Zeitgeber den nachfolgenden regenerierten Impuls empfängt, bis die spezifizierte Zeitdauer verstrichen ist.

4. System für die Messung der Zeitdauer der Arzneimittelinjektion in Echtzeit nach Anspruch 3, wobei die spezifizierte Zeitdauer ungefähr 300 Millisekunden beträgt.

5. System für die Messung der Zeitdauer der Arzneimittelinjektion in Echtzeit nach Anspruch 3 oder 4, wobei die Impulsklemmschaltung einen monostabilen Vibrator zum selektiven Umschalten zwischen einem stabilen Modus und einem nicht stabilen Modus umfasst, wobei in dem nicht stabilen Modus die Impulsklemmschaltung nicht auf nachfolgende Eingänge reagiert, bis die spezifizierte Zeitdauer abgelaufen ist.

6. System für die Messung der Zeitdauer der Arzneimittelinjektion in Echtzeit nach einem der Ansprüche 3-5, wobei die spezifizierte Zeitdauer eine längere Dauer als eine Dauer des ersten erfassten Schalls aufweist.

7. System für die Messung der Zeitdauer der Arzneimittelinjektion in Echtzeit nach einem der Ansprüche 1-6, wobei der Schallwandler einen Sensor umfasst.

8. Verfahren zum Messen in Echtzeit einer Injektionszeitdauer für eine Arzneimittelabgabevorrichtung (101), wobei das Verfahren Folgendes umfasst:
Erfassen, über einen Schallwandler (110), eines ersten Schalls, der der Einleitung der Arzneimittelabgabe der Arzneimittelabgabevorrichtung zugeordnet ist;
Umwandeln des ersten erfassten Schalls in eine erste elektronische Wellenform;
Umwandeln, über eine Wellenformkorrekturschaltung (120), die betriebsfähig mit dem Schallwandler gekoppelt ist, der ersten Wellenform in einen ersten Impuls;
Auslösen einer Impulsklemmschaltung (130), die betriebsfähig mit der Wellenformkorrekturschaltung gekoppelt ist, nach dem Empfangen des ersten Impulses;
Erzeugen, über die Impulsklemmschaltung, eines regenerierten Impulses;
Starten eines Zeitgebers (140), der betriebsfähig mit der Impulsklemmschaltung gekoppelt ist, nach dem Empfangen des regenerierten Impulses; und
Beenden des Zeitgebers nach dem Empfangen eines nachfolgenden regenerierten Impulses,
wobei eine Injektionszeit der Arzneimittelabgabevorrichtung basierend auf einer verstrichenen Zeit zwischen dem regenerierten Impuls, der von dem Zeitgeber empfangen wird, und dem nachfolgenden regenerierten Impuls gemessen wird.

9. Verfahren nach Anspruch 8, ferner umfassend die folgenden Schritte:
Erfassen, über den Schallwandler, eines zweiten Schalls, der dem Abschluss der Arzneimittelabgabe der Arzneimittelabgabevorrichtung zugeordnet ist;
Umwandeln des zweiten erfassten Schalls in eine zweite elektronische Wellenform;
Umwandeln der zweiten Wellenform in einen zweiten Impuls über die Wellenformkorrekturschaltung; und
Erzeugen des nachfolgenden regenerierten Impulses über die Impulsklemmschaltung.

10. Verfahren nach Anspruch 9, wobei der regenerierte Impuls, der von der Impulsklemmschaltung erzeugt wird, eine bestimmte Breite aufweist, die einer spezifizierten Zeitdauer entspricht, um zu verhindern, dass der Zeitgeber den nachfolgenden regenerierten Impuls empfängt, bis die spezifizierte Zeitdauer verstrichen ist.

11. Verfahren nach Anspruch 10, wobei die spezifizierte Zeitdauer ungefähr 300 Millisekunden beträgt.

12. Verfahren nach Anspruch 10 oder 11, wobei der Schritt des Auslösens der Impulsklemmschaltung ferner das selektive Umschalten, über einen monostabilen Vibrator, zwischen einem stabilen Modus und einem nicht stabilen Modus umfasst, wobei in dem nicht stabilen Modus die Impulsklemmschaltung nicht auf nachfolgende Eingänge reagiert, bis die spezifizierte Zeitdauer abgelaufen ist.

13. Verfahren nach einem der Ansprüche 10-12, wobei die spezifizierte Zeitdauer eine längere Dauer als eine Dauer des ersten erfassten Schalls aufweist.

14. Verfahren nach einem der Ansprüche 8-13, ferner umfassend den Schritt des Filterns, über die Wellenformkorrekturschaltung, mindestens eines hochfrequenten Signals, das durch die erste elektronische Wellenform und/oder die zweite elektronische Wellenform gebildet wird.

15. Verfahren nach einem der Ansprüche 8-14, wobei der Schallwandler einen Sensor umfasst.

## Revendications

1. Système de mesure de durée temporelle d'injection de médicament en temps réel (100), comprenant :
un transducteur sonore (110) adapté pour détecter un premier son associé à un démarrage d'administration de médicament d'un dispositif d'administration de médicament et un second son associé à un achèvement d'administration de médicament du dispositif d'administration de médicament, le transducteur sonore étant en outre adapté pour convertir les premier et second sons détectés en une première forme d'onde électronique et une seconde forme d'onde électronique, respectivement ;
un circuit de mise en forme de forme d'onde (120) couplé de façon opérationnelle au transducteur sonore, le circuit de mise en forme de forme d'onde étant adapté pour convertir les première et seconde formes d'onde en une première impulsion et une seconde impulsion, respectivement ;
un circuit de fixation de niveau d'impulsion (130) couplé de façon opérationnelle au circuit de mise en forme de forme d'onde, le circuit de fixation de niveau d'impulsion étant adapté pour se déclencher lors de la réception de la première impulsion et générer une impulsion régénérée ; et
une unité de mesure temporelle (140) couplée de façon opérationnelle au circuit de fixation de niveau d'impulsion, l'unité de mesure temporelle étant adaptée pour démarrer lors de la réception de l'impulsion régénérée et s'arrêter lors de la réception d'une impulsion subséquente régénérée par l'intermédiaire du circuit de fixation de niveau d'impulsion, dans lequel un temps d'injection du dispositif d'administration de médicament est mesuré sur la base d'un temps s'étant écoulé entre l'impulsion régénérée reçue par l'unité de mesure temporelle et l'impulsion subséquente régénérée.

2. Système de mesure de durée temporelle d'injection de médicament en temps réel de la revendication 1, dans lequel le circuit de mise en forme de forme d'onde est en outre adapté pour filtrer des signaux de hauteur aiguë générés par la première forme d'onde électronique et la seconde forme d'onde électronique.

3. Système de mesure de durée temporelle d'injection de médicament en temps réel de la revendication 1 ou 2, dans lequel le circuit de fixation de niveau d'impulsion est adapté pour générer une sortie d'impulsion ayant une largeur désignée correspondant à une durée spécifiée de temps, pour empêcher l'unité de mesure temporelle de recevoir l'impulsion subséquente régénérée avant qu'une durée spécifiée de temps ne se soit écoulée.

4. Système de mesure de durée temporelle d'injection de médicament en temps réel de la revendication 3, dans lequel la durée spécifiée de temps est d'approximativement 300 millisecondes.

5. Système de mesure de durée temporelle d'injection de médicament en temps réel de la revendication 3 ou 4, dans lequel le circuit de fixation de niveau d'impulsion comprend un vibrateur monostable adapté pour commuter sélectivement entre un mode stable et un mode non stable, dans lequel, dans le mode non stable, le circuit de fixation de niveau d'impulsion ne réagit pas à des entrées subséquentes avant l'achèvement de la durée spécifiée de temps.

6. Système de mesure de durée temporelle d'injection de médicament en temps réel de l'une quelconque des revendications 3 à 5, dans lequel la durée spécifiée de temps a une durée plus longue qu'une durée du premier son détecté.

7. Système de mesure de durée temporelle d'injection de médicament en temps réel de l'une quelconque des revendications 1 à 6, dans lequel le transducteur sonore comprend un capteur.

8. Procédé de mesure en temps réel d'une mesure de durée temporelle d'injection pour un dispositif d'administration de médicament (101), le procédé comprenant :
la détection, par l'intermédiaire d'un transducteur sonore (110), d'un premier son associé à un démarrage d'administration de médicament du dispositif d'administration de médicament ;
la conversion du premier son détecté en une première forme d'onde électronique ;
la conversion, par l'intermédiaire d'un circuit de mise en forme de forme d'onde (120) couplé de façon opérationnelle au transducteur sonore, de la première forme d'onde en une première impulsion ;
le déclenchement d'un circuit de fixation de niveau d'impulsion (130) couplé de façon opérationnelle au circuit de mise en forme de forme d'onde lors de la réception de la première impulsion ;
la génération, par l'intermédiaire du circuit de fixation de niveau d'impulsion, d'une impulsion régénérée ;
le démarrage d'une unité de mesure temporelle (140) couplée de façon opérationnelle au circuit de fixation de niveau d'impulsion lors de la réception de l'impulsion régénérée ; et
la terminaison de l'unité de mesure temporelle lors de la réception d'une impulsion subséquente régénérée,
dans lequel un temps d'injection du dispositif d'administration de médicament est mesuré sur la base d'un temps écoulé entre l'impulsion régénérée reçue par l'unité de mesure temporelle et l'impulsion subséquente régénérée.

9. Procédé de la revendication 8, comprenant en outre les étapes suivantes :
la détection, par l'intermédiaire du transducteur sonore, d'un second son associé à l'achèvement d'administration de médicament du dispositif d'administration de médicament ;
la conversion du second son détecté en une seconde forme d'onde électronique ;
la conversion de la seconde forme d'onde en une seconde impulsion par l'intermédiaire du circuit de mise en forme de forme d'onde ; et
la génération de l'impulsion subséquente régénérée par l'intermédiaire du circuit de fixation de niveau d'impulsion.

10. Procédé de la revendication 9, dans lequel l'impulsion régénérée générée par le circuit de fixation de niveau d'impulsion a une largeur désignée correspondant à une durée spécifiée de temps, pour empêcher l'unité de mesure de recevoir l'impulsion subséquente régénérée avant que la durée spécifiée de temps ne se soit écoulée.

11. Procédé de la revendication 10, dans lequel la durée spécifiée de temps d'approximativement 300 millisecondes.

12. Procédé de la revendication 10 ou 11, dans lequel l'étape du déclenchement du circuit de fixation de niveau d'impulsion comprend en outre la commutation sélective, par l'intermédiaire d'un vibrateur monostable, entre un mode stable et un mode non stable, dans lequel, dans le mode non stable, le circuit de fixation de niveau d'impulsion ne réagit pas à des entrées subséquentes avant l'achèvement de la durée spécifiée de temps.

13. Procédé de l'une quelconque des revendications 10 à 12, dans lequel la durée spécifiée de temps a une durée plus longue qu'une durée du premier son détecté.

14. Procédé de l'une quelconque des revendications 8 à 13, comprenant en outre l'étape du filtrage, par l'intermédiaire du circuit de mise en forme de forme d'onde, d'au moins un signal de hauteur aiguë généré par la première forme d'onde électronique et/ou la seconde forme d'onde électronique.

15. Procédé de l'une quelconque des revendications 8 à 14, dans lequel le transducteur sonore comprend un capteur.
